(19) Europäisches Patentamt
European Patent Office
Office européen des brevets

(11) **EP 4 252 862 A1**

(12) **EUROPEAN PATENT APPLICATION**
published in accordance with Art. 153(4) EPC

(43) Date of publication:
**04.10.2023 Bulletin 2023/40**

(21) Application number: **21898186.8**

(22) Date of filing: **30.11.2021**

(51) International Patent Classification (IPC):
**A61Q 17/00** *(2006.01)*          **A61K 8/37** *(2006.01)*
**A61K 8/41** *(2006.01)*          **A61K 8/49** *(2006.01)*
**A61K 8/72** *(2006.01)*          **A61K 8/92** *(2006.01)*

(52) Cooperative Patent Classification (CPC):
**A61K 8/02; A61K 8/37; A61K 8/41; A61K 8/49;
A61K 8/72; A61K 8/81; A61K 8/92; A61Q 17/00;
A61Q 17/04**

(86) International application number:
**PCT/JP2021/043804**

(87) International publication number:
**WO 2022/114215 (02.06.2022 Gazette 2022/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.11.2020 JP 2020198589**

(71) Applicant: **Kao Corporation**
**Chuo-ku**
**Tokyo 103-8210 (JP)**

(72) Inventors:
• **FUJIMOTO, Tatsuya**
**Tokyo 131-8501 (JP)**
• **HIRONO, Shingo**
**Tokyo 131-8501 (JP)**
• **ISHIDA, Kaori**
**Haga-gun, Tochigi 321-3497 (JP)**
• **KOBAYASHI, Hideo**
**Haga-gun, Tochigi 321-3497 (JP)**

(74) Representative: **Hoffmann Eitle**
**Patent- und Rechtsanwälte PartmbB**
**Arabellastraße 30**
**81925 München (DE)**

(54) **FILM-FORMING COMPOSITION**

(57) Provided is a composition having an excellent ultraviolet protection effect and being unlikely to cause uneven suntan even if the application is uneven. A film-forming composition contains components (a) and (b): (a) a nonvolatile oil containing an ultraviolet absorber; and (b) a fiber having an average fiber diameter of 0.1 $\mu$m or more and 7 $\mu$m or less in an amount of 0.5 mass% or more and 10 mass% or less in the film-forming composition, and the (average fiber diameter)$^2$/(fiber content) ($\mu$m$^2$/mass%) is 0.005 or more and 7 or less.

[Figure 1]

**Description**

Field of the Invention

The present invention relates to a film-forming composition.

Background of the Invention

Techniques for incorporating fibers into cosmetics are well known. For example, in order to stably disperse fine particles in cosmetics for a long time, it has been proposed to incorporate a fiber dispersion together with a solvent, the fiber dispersion composed of a thermoplastic polymer, having a number-average single fiber diameter of from 1 to 500 nm, and containing 60% or more in the sum Pa of the single fiber ratios (Patent Literature 1).
Patent Literature 2 discloses a fluid emulsion containing a polyamide fiber having a fiber length of from 0.3 to 1.5 mm and a thickness of 0.9 Dtex (fiber diameter: about 10.7 $\mu$m) , a UV-screening agent, and the like.

Citation List

Patent Literatures

Patent Literature 1: JP-A-2005-320506
Patent Literature 2: JP-A-2002-293731

Summary of the Invention

The present invention provides a film-forming composition comprising components (a) and (b):

(a) a nonvolatile oil containing an ultraviolet absorber; and

(b) a fiber having an average fiber diameter of 0.1 $\mu$m or more and 7 $\mu$m or less in an amount of 0.5 mass% or more and 10 mass% or less in the film-forming composition, in which

$$\text{(average fiber diameter)}^2 / \text{(fiber content)} \ (\mu m^2/mass\%) \ is$$

$$0.005 \ or \ more \ and \ 7 \ or \ less.$$

The present invention also provides a method for manufacturing a film on a skin surface, comprising applying the film-forming composition to the skin.
The present invention further provides a film comprising the film-forming composition.

Brief Description of Drawings

Figure 1 is a schematic diagram showing a structure of the electrostatic spraying device used for forming a fiber.
Figure 2 is a reference SEM image exemplifying the presence of network formation.
Figure 3 is a reference SEM image exemplifying the absence of network formation.
Figure 4 is an image showing the state of a film immediately after the application of the composition of Example 2.
Figure 5 is an image showing the state of a film after 15 minutes from the application of the composition of Example 2.

Detailed Description of the Invention

Despite application of a sunscreen cosmetic, uneven suntan can sometimes occur. There is a demand for suppressing such uneven suntan, but appropriate application is necessary for obtaining an ultraviolet protection effect without unevenness. For example, when a cosmetic is placed on a palm instead of direct placement on the application area, familiarized to both hands, and then applied or when a lower amount than appropriate is applied, it can cause uneven application, sometimes leading to undesired uneven suntan. A cosmetic containing a fiber having a thickness of 0.9 Dtex (fiber diameter: about 10.7 $\mu$m), such as that used in the fluid emulsion described in Patent Literature 2, together with a nonvolatile oil containing an ultraviolet absorber is required to appropriately applied in order to suppress uneven suntan.

The present invention relates to providing a composition having an excellent ultraviolet protection effect and being unlikely to cause uneven suntan even when the application is uneven.

The present inventors found that when a composition containing a combination of a nonvolatile oil containing an ultraviolet absorber and a fiber having an average fiber diameter of 0.1 $\mu$m or more and 7 $\mu$m or less at specific amounts with a specific range of (average fiber diameter)$^2$/(fiber content) ($\mu$m$^2$/mass%) is applied to a skin and it is dried, the nonvolatile oil containing an ultraviolet absorber spreads over the application area and forms a highly uniform film, the ultraviolet protection effect is excellent, and even if it is applied unevenly, uneven suntan is unlikely to occur, and the present invention was accomplished.

The film-forming composition of the present invention has an excellent ultraviolet protection effect and is unlikely to cause uneven suntan even if it is applied unevenly.

<Component (a)>

The film-forming composition of the present invention contains (a) a nonvolatile oil containing an ultraviolet absorber.

In the present invention, the "ultraviolet absorber (hereinafter, also referred to as component (a1))" may be any oil-soluble ultraviolet absorber and is a concept encompassing an oil-soluble ultraviolet absorber in a liquid state at 25°C and 1 atm, an oil-soluble ultraviolet absorber in a solid state at 25°C and 1 atm, and a combination thereof. The term "oil-soluble ultraviolet absorber" means an ultraviolet absorber having a solubility in water of less than 0.01 mass%. In the present invention, it is preferable to at least use an oil-soluble ultraviolet absorber in a liquid state at 25°C and 1 atm as an ultraviolet absorber. In this case, an oil-soluble ultraviolet absorber in a solid state at 25°C and 1 atm may be used in combination with an oil-soluble ultraviolet absorber in a liquid state at 25°C and 1 atm.

In the present invention, the "nonvolatile oil containing an ultraviolet absorber" may be any nonvolatile oil at least containing an oil-soluble ultraviolet absorber as described above and is preferably a nonvolatile liquid oil containing an ultraviolet absorber. Here, the nonvolatile liquid oil containing an ultraviolet absorber refers to a nonvolatile oil at least containing an oil-soluble ultraviolet absorber in liquid form, and when the nonvolatile liquid oil containing an ultraviolet absorber is used, the film-forming composition of the present invention encompasses, for example, an aspect in which the nonvolatile oil contained is only an oil-soluble ultraviolet absorber in a liquid state at 25°C and 1 atm, an aspect in which the nonvolatile oil contained is a combination of an oil-soluble ultraviolet absorber in a liquid state at 25°C and 1 atm and one or more selected from a nonvolatile oil (a2) (hereinafter, also referred to as "additional oil") other than the oil-soluble ultraviolet absorber and an oil-soluble ultraviolet absorber in a solid state at 25°C and 1 atm, and an aspect in which the nonvolatile oil contained is a combination of an oil-soluble ultraviolet absorber in a solid state at 25°C and 1 atm and an additional liquid oil.

The total content of the component (a) in the film-forming composition of the present invention is preferably 0.5 mass% or more, more preferably 2.5 mass% or more, further more preferably 5 mass% or more, further more preferably 7.5 mass% or more, and particularly preferably 10 mass% or more from the viewpoint of e.g., the uniformity of the film, and the total content in the film-forming composition of the present invention is preferably 90 mass% or less, more preferably 65 mass% or less, further more preferably 45 mass% or less, and particularly preferably 40 mass% or less from the viewpoint of e.g., the use impression (no oily and sticky feelings). The specific range is preferably 0.5 mass% or more and 90 mass% or less, more preferably 2.5 mass% or more and 65 mass% or less, further more preferably 5 mass% or more and 45 mass% or less, further more preferably 7.5 mass% or more and 45 mass% or less, and particularly preferably 10 mass% or more and 40 mass% or less in the film-forming composition of the present invention.

The total content of the component (a) can be measured, for example, by identifying the skeleton structure of each oil molecule by known technology such as NMR, chromatography, and IR analysis or a combination thereof and determining based on the intensity of the measured value of a portion showing the skeleton structure.

The content of the ultraviolet absorber in the film-forming composition of the present invention is preferably 0.1 mass% or more, more preferably 1 mass% or more, further more preferably 3 mass% or more, and particularly preferably 7 mass% or more from the viewpoint of e.g., the ultraviolet protection effect, the uneven suntan prevention effect, the uniformity of the film, and the content of the ultraviolet absorber in the film-forming composition of the present invention is preferably 55 mass% or less, more preferably 50 mass% or less, further more preferably 45 mass% or less, and particularly preferably 40 mass% or less from the viewpoint of e.g., the use impression (no oily and sticky feelings). The specific range is preferably 0.1 mass% or more and 55 mass% or less, more preferably 1 mass% or more and 50 mass% or less, further more preferably 3 mass% or more and 45 mass% or less, and particularly preferably 7 mass% or more and 40 mass% or less in the film-forming composition of the present invention.

When the content of the ultraviolet absorber is 7 mass% or more, the ultraviolet protection effect, the uneven suntan prevention effect, and the uniformity of the film are particularly improved.

The mass ratio of the component (a1) ultraviolet absorber to the total of the component (a) nonvolatile oil (when the additional oil (a2) is used in addition to the ultraviolet absorber, the total of the ultraviolet absorber and the additional oil), (a1)/(a), is preferably 0.1 or more, more preferably 0.3 or more, further more preferably 0.5 or more, and particularly preferably 0.7 or more from the viewpoint of e.g., the ultraviolet protection effect, the uneven suntan prevention effect, the uniformity of the film, and is preferably 1 or less from the viewpoint of e.g., the solubility of the ultraviolet absorber, the use impression. The specific range is preferably 0.1 or more and 1 or less, more preferably 0.3 or more and 1 or less, further more preferably 0.5 or more and 1 or less, and particularly preferably 0.7 or more and 1 or less.

When the mass ratio (a1)/(a) is adjusted to 0.5 or more, the ultraviolet protection effect, the uneven suntan prevention effect, and the uniformity of the film are particularly improved.

(Component (a1))

Examples of the ultraviolet absorber include a benzoic acid-based ultraviolet absorber, an anthranilic acid-based ultraviolet absorber, a salicylic acid-based ultraviolet absorber, a cinnamic acid-based ultraviolet absorber, a benzoylmethane-based ultraviolet absorber, a triazine-based ultraviolet absorber, a benzophenone-based ultraviolet absorber, and a hydantoin-based ultraviolet absorber. Among them, one or more selected from the group consisting of benzoic acid-based ultraviolet absorbers, cinnamic acid-based ultraviolet absorbers, and triazine-based ultraviolet absorbers are preferable from the viewpoint of e.g., the ultraviolet protection effect.

The ultraviolet absorbers may be used singly or in combinations of two or more thereof.

Examples of the benzoic acid-based ultraviolet absorber include para-aminobenzoic acid, glyceryl para-aminobenzoate, ethyl dihydroxypropyl para-aminobenzoate, octyl dimethyl para-aminobenzoate, amyl para-dimethylaminobenzoate, and diethylamino hydroxybenzoyl hexyl benzoate.

Examples of the anthranilic acid-based ultraviolet absorber include methyl anthranilate.

Examples of the salicylic acid-based ultraviolet absorber include homomenthyl salicylate, 2-ethylhexyl salicylate, and triethanolamine salicylate.

Examples of the cinnamic acid-based ultraviolet absorber include 2-ethylhexyl para-methoxycinnamate, glyceryl mono-2-ethylhexanoate di-para-methoxycinnamate, methyl 2,5-diisopropylcinnamate, methylbis (trimethylsiloxy)silylisopentyl trimethoxycinnamate, isopropyl para-methoxycinnamate, a mixture of isopropyl para-methoxycinnamate and diisopropyl cinnamate, 2-ethoxyethyl para-methoxycinnamate, and diethanolamine para-methoxycinnamate.

Examples of the benzoylmethane-based ultraviolet absorber include 4-isopropyldibenzoylmethane and 4-tert-butyl-4'-methoxydibenzoylmethane.

Examples of the triazine-based ultraviolet absorber include 2,4,6-tris[4-(2-ethylhexyloxycarbonyl)anilino]-1,3,5-triazine and 2,4-bis-[{4-(2-ethylhexyloxy)-2-hydroxy}-phenyl]-6-(4-methoxyphenyl)-1,3,5-triazine.

Examples of the benzophenone-based ultraviolet absorber include 2,4-dihydroxybenzophenone, 2,2'-dihydroxy-4-methoxybenzophenone, 2-hydroxy-4-methoxybenzophenone, 2,2'-dihydroxy-4,4'-dimethoxybenzophenone, 2,2'4,4'-tetrahydroxybenzophenone, 4-(2-β-glucopyranosiloxy)propoxy-2-hydroxybenzophenone, and 2-hydroxy-4-n-octyloxybenzophenone.

Examples of the hydantoin-based ultraviolet absorber include 2-ethylhexyl dimethoxybenzylidene dioxoimidazolidine propionate.

Other examples of the ultraviolet absorber include octocrylene, cinoxate, phenylbenzimidazole sulfonic acid, 1-(3,4-dimethoxyphenyl)-4,4-dimethyl-1,3-pentanedione, 3-(4-methylbenzylidene)camphor, and methylene bis-benzotriazolyl tetramethylbutylphenol.

For example, the above-mentioned diethylaminohydroxybenzoyl hexyl benzoate, 2,4,6-tris[4-(2-ethylhexyloxycarbonyl)anilino]-1,3,5-triazine, 2,4-bis-[{4-(2-ethylhexyloxy)-2-hydroxy}-phenyl]-6-(4-methoxyphenyl)-1,3,5-triazine are oil-soluble ultraviolet absorbers in a solid state at 25°C and 1 atm. In contrast, the above-mentioned 2-ethylhexyl para-methoxycinnamate, isopropyl para-methoxycinnamate, and 2-ethoxyethyl para-methoxycinnamate are oil-soluble ultraviolet absorbers in a liquid state at 25°C and 1 atm.

(Component (a2))

The film-forming composition of the present invention may contain (a2) a nonvolatile oil (additional oil) other than an oil-soluble ultraviolet absorber in addition to the ultraviolet absorber.

The additional oil is, for example, one or more oils selected from the group consisting of ester oils, ether oils, hydrocarbon oils, higher alcohols, silicone oils, and fluorine oils, and is preferably one or more oils selected from the group consisting of ester oils, ether oils, hydrocarbon oils, and higher alcohols and more preferably one or more oils selected from the group consisting of ester oils, ether oils, and hydrocarbon oils, from the viewpoint of e.g., the solubility of the ultraviolet absorber, the use impression.

The additional oil preferably has an IOB value of higher than 0 and 0.6 or less and more preferably an IOB value of 0.05 or more and 0.3 or less.

The IOB value is an abbreviation of inorganic/organic balance, a value showing the ratio of the inorganic value to the organic value, and is an indicator showing the degree of polarity of an organic compound. The IOB value is specifically represented as "IOB value = inorganic value/organic value". Here, an "inorganic value" and an "organic value" are respectively set in accordance with various atoms or functional groups, for example, such that the "organic value" for one carbon atom in a molecule is 20, and the "inorganic value" for one hydroxy group in a molecule is 100. The IOB value of an organic compound can be calculated by adding up the "inorganic values" and the "organic values" of all atoms and functional groups in the organic compound (see, for example, written by Fujita, "Kagaku no ryoiki (Journal of Japanese chemistry)" vol. 11, No. 10, pp. 719-725, 1957).

Examples of the ester oil include one or more selected from the group consisting of esters composed of a linear or branched fatty acid and a linear or branched alcohol or polyhydric alcohol and triglyceryl fatty acid esters (triglycerides).

Specifically, the ester oil may be one or more selected from the group consisting of isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, n-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, pentaerythrityl tetra-2-ethylhexanoate, glyceryl tri-2-ethylhexanoate, trimethylolpropane triisostearate, cetyl 2-ethylhexanoate, 2-ethylhexyl palmitate, diethylhexyl naphthalenedicarboxylate, C12-15 alkyl benzoate, cetearyl isononanoate, glyceryl tri(caprylate/caprate), butylene glycol (dicaprylate/caprate), glyceryl trilaurate, glyceryl trimyristate, glyceryl tripalmitate, glyceryl triisostearate, glyceryl tri-2-heptylundecanoate, glyceryl tribehenate, glyceryl tricocoate, castor oil fatty acid methyl ester, oleyl oleate, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, di-2-ethylhexyl succinate, triethyl citrate, and tripropylene glycol dipivalate.

Among the ester oils, from the viewpoint of e.g., the solubility of the ultraviolet absorber, the use impression, preferred are one or more selected from the group consisting of octyldodecyl myristate, myristyl myristate, isocetyl stearate, isocetyl isostearate, cetearyl isononanoate, diisobutyl adipate, di-2-ethylhexyl sebacate, isopropyl myristate, isopropyl palmitate, diisostearyl malate, neopentyl glycol dicaprate, C12-15 alkyl benzoate, and glyceryl tri(caprylate/caprate), more preferred are one or more selected from the group consisting of isopropyl myristate, isopropyl palmitate, diisostearyl malate, neopentyl glycol dicaprate, C12-15 alkyl benzoate, and glyceryl tri(caprylate/caprate), further more preferred are one or more selected from the group consisting of isopropyl myristate, neopentyl glycol dicaprate, C12-15 alkyl benzoate, and glyceryl tri(caprylate/caprate), and particularly preferred are one or two selected from the group consisting of neopentyl glycol dicaprate and C12-15 alkyl benzoate.

Examples of the ether oil include, in addition to alkyl-1,3-dimethylbutyl ether such as cetyl dimethylbutyl ether, ethylene glycol dioctyl ether, glycerol monooleyl ether, and dicaprylyl ether. These may be used alone or in combination of two or more. Among the ether oils, dicaprylyl ether is preferable.

Examples of the hydrocarbon oil include hydrocarbon oils in a liquid state at 20°C, such as squalane, squalene, n-heptane, n-octane, cyclohexane, liquid paraffin, light isoparaffin, and liquid isoparaffin; and hydrocarbon oils in a solid or semisolid state at 20°C, such as Vaseline, ceresin, paraffin wax, microcrystalline wax, ozokerite, hydrogenated polyisobutene, polyethylene wax, and polyolefin wax. These may be used alone or in combination of two or more. Among the hydrocarbon oils, preferred are one or more selected from the group consisting of squalane, liquid paraffin, light isoparaffin, liquid isoparaffin, Vaseline, cerecin, paraffine wax, microcrystalline wax, polyethylene wax, and polyolefin wax, and more preferred are one or more selected from the group consisting of squalane, liquid paraffin, and light isoparaffin.

The hydrocarbon oil in a liquid, solid, or semisolid state at 20°C as described above is preferably a hydrocarbon oil satisfying at least either the condition of a boiling point at atmospheric pressure of 260°C or more or the condition of a vapor pressure at 25°C of 0.02 mmHg or less, more preferably a hydrocarbon oil having a vapor pressure at 25°C of 0.01 mmHg or less, and further more preferably a hydrocarbon oil having a vapor pressure lower than that of water.

The higher alcohol is preferably an alcohol having 12 to 20 carbon atoms and specifically may be one or more selected from the group consisting of cetyl alcohol, stearyl alcohol, isostearyl alcohol, and oleyl alcohol.

Animal and vegetable oils including an ester oil and a hydrocarbon oil may be used as the additional oil. Examples of the animal and vegetable oils include olive oil, jojoba oil, macadamia nut oil, meadowfoam oil, castor oil, safflower oil, sunflower oil, avocado oil, canola oil, apricot kernel oil, rice germ oil, and rice bran oil. These may be used alone or in combination of two or more. Among the animal and vegetable oils, olive oil is preferable.

Examples of the silicone oil include methylpolysiloxane, methylphenylpolysiloxane, polyether-modified silicone, amino-modified silicone, carboxy-modified silicone, fatty acid ester-modified silicone, alcohol-modified silicone, epoxy-modified silicone, fluorine-modified silicone, and alkyl-modified silicone. These may be used alone or in combination of two or more.

Examples of the fluorine oil include perfluorodecaline, perfluoroadamantane, perfluorobutyl tetrahydrofuran, perfluoropentane, perfluorooctane, perfluorononane, perfluorodecane, perfluorododecane, and perfluoropolyether. These may be used alone or in combination of two or more.

<Component (b)>

The component (b) is a fiber having an average fiber diameter of 0.1 μm or more and 7 μm or less. When the average fiber diameter is less than 0.1 μm, aggregation is likely to occur. When the average fiber diameter exceeds 7 μm, network is not formed in the film, and the uniformity of the film and the uneven suntan prevention effect become insufficient.

The average fiber diameter is preferably 0.2 μm or more, more preferably 0.3 μm or more, and further more preferably 0.4 μm or more from the viewpoint of e.g., the uniformity of the film, the uneven suntan prevention effect, the difficulty in aggregation, and is preferably 5 μm or less, more preferably 4 μm or less, further more preferably 3 μm or less, further more preferably 1.5 μm or less, and particularly preferably 0.8 μm or less from the viewpoint of e.g., the uniformity of the film, the uneven suntan prevention effect, the ease of network formation, the ultraviolet protection effect. The specific range is preferably 0.2 μm or more and 5 μm or less, more preferably 0.3 μm or more and 4 μm or less, further more preferably 0.4 μm or more and 3 μm or less, further more preferably 0.4 μm or more and 1.5 μm or less, and particularly preferably 0.4 μm or more and 0.8 μm or less.

The average fiber diameter is generally the diameter of a cross-section of a fiber. That is, when the cross-section of a fiber is a circle, the average fiber diameter is determined on the basis of the diameter, and when the cross-section is an ellipse, the average fiber diameter is determined on the basis of the long diameter.

The average fiber diameter can be determined as follows. That is, observation with an SEM is performed by magnifying the fibers 2 000 times or 5 000 times, and from the two-dimensional image thereof, 100 fibers excluding defects (e.g., clumps of fibers and crossing parts of fibers) are arbitrarily selected. A line perpendicular to the longitudinal direction of each of the fibers is drawn, and the fiber diameters are directly read as measured values and are arithmetically averaged to determine the average fiber diameter. When fibers are dispersed in a film-forming composition, the film-forming composition is thinly applied to a substrate, and measurement by SEM observation is performed.

The average fiber length of the component (b) is preferably 1 μm or more, more preferably 3 μm or more, further more preferably 20 μm or more, further more preferably 25 μm or more, further more preferably 30 μm or more, and particularly preferably 40 μm or more from the viewpoint of e.g., the ease of network formation, the uniformity of the film, the uneven suntan prevention effect, the durability of the film, the adhesion, and is preferably 300 μm or less, more preferably 250 μm or less, further more preferably 200 μm or less, and particularly preferably 150 μm or less from the viewpoint of e.g., the difficulty in aggregation. The specific range is preferably 1 μm or more and 300 μm or less, more preferably 3 μm or more and 300 μm or less, further more preferably 20 μm or more and 300 μm or less, further more preferably 25 μm or more and 250 μm or less, further more preferably 30 μm or more and 200 μm or less, and particularly preferably 40 μm or more and 150 μm or less.

The average fiber length can be determined as follows. That is, observation with an SEM is performed by magnifying the fibers 250 times to 750 times in accordance with the length of the fibers, and from the two-dimensional image thereof, 100 fibers excluding defects (e.g., clumps of fibers and crossing parts of fibers) are arbitrarily selected. A line is drawn in the longitudinal direction of each of the fibers, and the fiber lengths are directly read as measured values and are arithmetically averaged to determine the average fiber length. When fibers are dispersed in a film-forming composition, the film-forming composition is thinly applied to a substrate, and measurement by SEM observation is performed.

The aspect ratio (average fiber length/average fiber diameter) of the fiber of the component (b) is preferably 8 or more, more preferably 10 or more, further more preferably 15 or more, further more preferably 20 or more, further more preferably 30 or more, and particularly preferably 60 or more from the viewpoint of e.g., the uniformity of the film, the uneven suntan prevention effect, and is preferably 300 or less, more preferably 250 or less, further more preferably 200 or less, and particularly preferably 150 or less from the viewpoint of e.g. the uniformity of the film, the uneven suntan prevention effect, the difficulty in aggregation. The specific range is preferably 8 or more and 300 or less, more preferably 10 or more and 250 or less, further more preferably 15 or more and 200 or less, further more preferably 20 or more and 150 or less, further more preferably 30 or more and 150 or less, and particularly preferably 60 or more and 150 or less.

When the aspect ratio (average fiber length/average fiber diameter) of the fiber of the component (b) is 15 or more, the ultraviolet protection effect, the uneven suntan prevention effect, and the uniformity of the film are particularly improved.

The CV (coefficient of variation) value of the fiber length of the component (b) is preferably 40% or more, more preferably 42% or more, and further preferably 45% or more from the viewpoint of e.g., the ease of network formation, the uniformity of the film, the uneven suntan prevention effect, and is preferably 100% or less, more preferably 95% or less, and further more preferably 90% or less from the viewpoint of e.g., the ease of network formation, the uniformity of the film, the uneven suntan prevention effect, the storage stability. The specific range is preferably 40% or more and 100% or less, more preferably 42% or more and 95% or less, and further preferably 45% or more and 90% or less.

The CV value is a value obtained by calculating the (standard deviation of measured fiber lengths)/(average fiber length) $\times$ 100 (%) from the measured values of the fiber length obtained by the above-described measuring method.

In the fiber of the component (b), the rate of the number of fibers having a fiber length of 40 $\mu$m or more to the total number of the fibers is preferably 5% or more and 100% or less, more preferably 8% or more and 100% or less, and further preferably 15% or more and 100% or less from the viewpoint of e.g., the ease of network formation, the uniformity of the film, the uneven suntan prevention effect.

The above-mentioned rate of the number of fibers is calculated by adjusting the magnification of the SEM within $\times$200 to $\times$750 in accordance with the fiber length such that 20 to 30 fibers are present in one imaging screen of the SEM and, in this state, measuring the fiber lengths of all fibers in the image for eliminating arbitrariness. The rate is determined by measuring 200 or more fibers in total.

The fiber of the component (b) is preferably a water-insoluble polymer fiber from the viewpoint of e.g., the ease of network formation, the uniformity of the film, the uneven suntan prevention effect.

Here, the term "water-insoluble polymer fiber" refers to a fiber having a property that when 1 g of the fiber is weighed in an environment of 23°C and 1 atm and is then immersed in 10 g of deionized water for 24 hours, the dissolved amount of the immersed fiber does not exceed 0.5 g.

The fiber of the component (b) can be manufactured by, for example, obtaining a fiber from a fiber-forming polymer by various known spinning technologies and subjecting the fiber to shortening treatment.

Here, the fiber-forming polymer is usually a thermoplastic or solvent-soluble chain polymer molecule and is preferably a thermoplastic resin and more preferably a thermoplastic resin having a weight-average molecular weight of 10 000 or more and 200 000 or less. The fiber-forming polymer is preferably a water-insoluble polymer in order to maintain the form of the fiber in the film-forming composition. The spinning method is preferably an electrospinning method in order to efficiently obtain a fiber having a small fiber diameter.

Examples of the water-insoluble polymer include polyvinyl alcohols, such as a completely saponified polyvinyl alcohol that can be insolubilized after film formation and a partially saponified polyvinyl alcohol that can be crosslinked after film formation by using a crosslinking agent together; oxazoline-modified silicone, such as a poly(N-propanoyleth-yleneimine) graft-dimethylsiloxane/γ-aminopropylmethylsiloxane copolymer; biodegradable resins, such as polyvinylacetal diethylaminoacetate, Zein (major component of corn protein), polylactic acid (PLA), polybutylene succinate, polyglycolic acid, polycaprolactone, and polyhydroxy alkanoic acid; polyester resins, such as polylactic acid (PLA), polyethylene terephthalate (PET), and polybutylene terephthalate; (meth)acrylic resins of polymers containing one or more structural units selected from the group consisting of a structural unit derived from (meth)acrylic acid or a salt thereof, a structural unit derived from (meth)acrylic acid ester, a structural unit derived from (meth)acrylonitrile, a structural unit derived from (meth)acrylamide, and a structural unit derived from N-substituted (meth)acrylamide; water-insoluble polysaccharides, such as cellulose, chitin, and chitosan; polyamide resins, such as Nylon; and a polyimide resin, polyamideimide resin, a polystyrene resin, a polyvinylbutyral resin, a polyvinylacetal resin, a polyurethane resin, a polypropylene resin, a polyethylene resin, and various polypeptides (e.g., collagen, gelatin, fibrin, and casein). These water-insoluble polymers can be used alone or in combination of two or more.

Among these water-insoluble polymers, preferred are one or more selected from the group consisting of an oxazoline-modified silicone, a biodegradable resin (more preferably polyvinylacetal diethylaminoacetate, Zein, or polylactic acid), a polyester resin, a (meth)acrylic resin, a water-insoluble polysaccharide, a polyamide resin, a completely saponified polyvinyl alcohol that can be insolubilized after film formation, a partially saponified polyvinyl alcohol that can be crosslinked after film formation by using a crosslinking agent together, a polyvinylbutyral resin, a polyurethane resin, and a polypropylene resin from the viewpoint of e.g., the ease of network formation, the uniformity of the film, the uneven suntan prevention effect, more preferred are one or more selected from the group consisting of a polyester resin, a (meth)acrylic resin, a water-insoluble polysaccharide, a polyamide resin, a polyvinylbutyral resin, a polyurethane resin, and a polypropylene resin from the viewpoint of the ease in formation of nanofibers, further more preferred are one or more selected from the group consisting of a polyester resin, a (meth)acrylic resin, a water-insoluble polysaccharide, and a polyamide resin, further more preferred are one or more selected from a polyester resin and a (meth)acrylic resin, and particularly preferred is a (meth)acrylic resin.

The (meth)acrylic resin is preferably an (alkyl (meth)acrylamide/hydroxyalkyl (meth)acrylate/alkylaminoalkyl (meth)acrylate) copolymer and more preferably an (octyl (meth)acrylamide/hydroxypropyl (meth)acrylate/butylaminoethyl (meth)acrylate) copolymer.

In addition, it is also preferable from the viewpoint of the reduction in environmental load to use a biodegradable resin such as polylactic acid, polybutylene succinate, polyglycolic acid, polycaprolactone, or polyhydroxy alkanoic acid.

In the present specification, the term "biodegradability" means that the degree of biodegradation measured in accordance with JIS K6953-1 is 30% or more.

The cellulose is preferably a modified cellulose fiber having a cellulose type I crystal structure and including a modifying group bound to the cellulose fiber. The modifying group is preferably bound to a part or all of the hydroxy groups of the cellulose fiber or to the carboxy group converted from the group ($-CH_2OH$) at the C6 position of a glucose unit.

The modifying group preferably contains one or more selected from the group consisting of (a) a hydrocarbon group, (b) a silicone chain, and (c) an alkylene oxide chain. The cellulose fiber to which a modifying group binds is preferably an anion-modified cellulose fiber from the viewpoint of the ease in binding of a modifying group.

Examples of the means for the fiber shortening treatment include cutting, shearing, crushing, pulverizing, disintegrating, and fibrillating. Examples of the means include dry grinders, for example, impact crushers such as a mechanical vortex crusher and a hammer crusher, jet grinders such as a jet mill, medium grinders such as a ball mill and a rod mill, cutter mill grinders, and disc mill grinders; and media grinders using liquid media and wet grinders using medialess grinders, and a combination thereof can also be used. In the means for the fiber shortening treatment, it is preferable to manufacture a fiber assembly in which nanofibers are entangled (examples thereof include nonwoven fabrics, encompassing those having a prescribed thickness, such as flocculate), cut the fiber assembly into an appropriate size, and then perform fiber shortening treatment using a mechanical vortex grinder, a cutter mill grinder, a disc mill grinder, a wet high-speed shear medialess grinder, or a wet high-pressure shear medialess grinder.

The content of the fiber of the component (b) in the film-forming composition is 0.5 mass% or more and 10 mass% or less. When the content of the fiber of the component (b) is less than 0.5 mass%, network is not formed in the film, and the uniformity of the film and the uneven suntan prevention effect become insufficient. When the content of the fiber of the component (b) exceeds 10 mass%, the uniformity of the film and the uneven suntan prevention effect become insufficient.

The content of the fiber of the component (b) in the film-forming composition of the present invention is preferably 0.7 mass% or more, more preferably 1 mass% or more, further more preferably 2 mass% or more, and particularly preferably 3 mass% or more from the viewpoint of e.g., the ease of network formation, the uniformity of the film, the uneven suntan prevention effect, the ultraviolet protection effect, and the content in the film-forming composition of the present invention is preferably 9 mass% or less, more preferably 8.5 mass% or less, further more preferably 8 mass% or less, further more preferably 6 mass% or less, and particularly preferably 5 mass% or less from the viewpoint of e.g., the uniformity of the film, the uneven suntan prevention effect, the ultraviolet protection effect, the difficulty in aggregation, the use impression. The specific range in the film-forming composition of the present invention is preferably 0.7 mass% or more and 9 mass% or less, more preferably 1 mass% or more and 8.5 mass% or less, further more preferably 2 mass% or more and 8 mass% or less, further more preferably 3 mass% or more and 6 mass% or less, and particularly preferably 3 mass% or more and 5 mass% or less.

The content of the fiber of the compound (b) based on the total mass of the film-forming composition can be determined as follows. For example, when the fiber of the component (b) is a water-insoluble polymer, fibers of the water-insoluble polymers are collected from the fibers contained in the film-forming composition. Subsequently, the fibers are washed with a solvent in which the fibers are not soluble, and only the fibers of the water-insoluble polymer are obtained by filtering. When the fiber of the component (b) is a fiber of a polyester resin such as polylactic acid, the solvent is preferably ethanol, and when the fiber of the component (b) is a fiber of a (meth)acrylic resin, the solvent is preferably water. The mass of the thus-collected fiber of the water-insoluble polymer is measured, and the content of the fiber of the component (b) can be determined by the following expression:

```
(Content of fiber of component (b) (mass%)) = (mass
of fiber of component (b) after washing)/(mass of
composition before washing (i.e., film-forming
composition)) × 100.
```

The mass ratio of the component (b) to the component (a), (b)/(a), is preferably 0.005 or more, more preferably 0.02 or more, further more preferably 0.05 or more, and particularly preferably 0.1 or more from the viewpoint of e.g., the uniformity of the film, the uneven suntan prevention effect, the durability of the film, the adhesion, and is preferably 5 or less, more preferably 4 or less, further more preferably 3 or less, further more preferably 2 or less, further more preferably 0.4 or less, further more preferably 0.25 or less, and particularly preferably 0.2 or less from the viewpoint of e.g., the uniformity of the film, the uneven suntan prevention effect, the difficulty in aggregation, the difficulty in occurrence of twisting. The specific range is preferably 0.005 or more and 5 or less, more preferably 0.02 or more and 4 or less, further more preferably 0.05 or more and 3 or less, further more preferably 0.1 or more and 2 or less, further more preferably 0.1 or more and 0.4 or less, further more preferably 0.1 or more and 0.25 or less, and particularly preferably 0.1 or more and 0.2 or less.

In the film-forming composition of the present invention, (average fiber diameter)$^2$/(fiber content) ($\mu m^2$/mass%) of the component (b) is 0.005 or more and 7 or less. When the (average fiber diameter)$^2$/(fiber content) ($\mu m^2$/mass%) is less than 0.005 or exceeds 7, network is not formed in the film, and the uniformity of the film and the uneven suntan prevention effect become insufficient.

The (average fiber diameter)$^2$/(fiber content) ($\mu m^2$/mass%) of the component (b) is preferably 0.01 or more, more preferably 0.02 or more, further more preferably 0.03 or more, and particularly preferably 0.05 or more from the viewpoint of e.g., the uniformity of the film, the uneven suntan prevention effect, the difficulty in aggregation, the ultraviolet protection effect, and is preferably 6 or less, more preferably 5 or less, further more preferably 4 or less, further more preferably 3 or less, further more preferably 1 or less, further more preferably 0.5 or less, further more preferably 0.2 or less, and particularly preferably 0.1 or less from the viewpoint of e.g., the uniformity of the film, the uneven suntan prevention effect, the ultraviolet protection effect. The specific range is preferably 0.01 or more and 6 or less, more preferably 0.02 or more and 5 or less, further more preferably 0.03 or more and 4 or less, further more preferably 0.05 or more and 3 or less, further more preferably 0.05 or more and 1 or less, further more preferably 0.05 or more and 0.5 or less, further more preferably 0.05 or more and 0.2 or less, and particularly preferably 0.05 or more and 0.1 or less.

The fiber content means the content (mass%) of the fiber of the component (b) based on the total mass of the film-forming composition. The value of (average fiber diameter)$^2$/(fiber content) ($\mu m^2$/mass%) is an indicator of the cumulative length of the fiber contained in the film-forming composition and means that the larger this value, the shorter the cumulative length.

The film-forming composition of the present invention preferably further contains a volatile component, in addition to the components (a) and (b), from the viewpoint of e.g., the ease of network formation, the uniformity of the film, the uneven suntan prevention effect, the use impression.

The volatile component is, for example, one or more selected from the group consisting of water, alcohol, amides, ketone, and volatile silicone oil and is preferably one or more selected from the group consisting of water, alcohol, and volatile silicone oil.

Examples of the alcohol include a chain aliphatic monohydric alcohol, a cyclic aliphatic monohydric alcohol, and an aromatic monohydric alcohol.

The chain aliphatic monohydric alcohol preferably has 1 to 6 carbon atoms, the cyclic aliphatic monohydric alcohol preferably has 4 to 6 carbon atoms, and the aromatic monohydric alcohol is preferably benzyl alcohol or phenylethyl alcohol.

Appropriate examples of the alcohol include ethanol, n-propanol, isopropyl alcohol, butyl alcohol, n-pentanol, and phenylethyl alcohol. Among them, ethanol is preferable from the viewpoint of safety. The alcohols may be used singly or in combinations of two or more thereof.

Examples of the volatile silicone oil include volatile methylpolysiloxane and volatile cyclic silicone.

The content of the volatile component in the film-forming composition of the present invention is preferably 20 mass% or more, more preferably 30 mass% or more, further more preferably 45 mass% or more, and particularly preferably 50 mass% or more from the viewpoint of e.g., the use impression, and the content in the film-forming composition of the present invention is preferably 99 mass% or less, more preferably 95 mass% or less, further more preferably 90 mass% or less, and particularly preferably 85 mass% or less from the viewpoint of e.g., the uniformity of the film, the uneven suntan prevention effect, the ultraviolet protection effect. The specific range in the film-forming composition of the present invention is preferably 20 mass% or more and 99 mass% or less, more preferably 30 mass% or more and 95 mass% or less, further more preferably 45 mass% or more and 90 mass% or less, and particularly preferably 50 mass% or more and 85 mass% or less.

The film-forming composition of the present invention may contain, for example, a powder, a surfactant, an emulsion stabilizer, a polyol in a liquid state at 20°C, a water-soluble polymer, a preservative, a pH adjuster, a moisturizing agent, an algefacient, an amino acid, a perfume, and a metal sequestering agent, in addition to the above-described components. These may be used alone or in combination of two or more.

The powder is roughly divided into ultraviolet scattering agents and powder other than the ultraviolet scattering agents (e.g., organic powder, inorganic powder, and organic-inorganic composite powder). These may be used alone or in combination of two or more. The shape of the powder is not particularly limited, and examples thereof include spherical, plate-like, rod-like, spindle-like, needle-like, and amorphous.

The ultraviolet scattering agent is preferably a hydrophobized ultraviolet scattering agent and more preferably a hydrophobized metal oxide fine particle.

The metal oxide fine particle used as the hydrophobized metal oxide fine particle is preferably one or more metal oxide fine particles selected from the group consisting of zinc oxide, titanium oxide, cerium oxide, iron oxide, and chromium oxide from the viewpoint of availability. These metal oxide fine particles can contain trace elements of +2 valence or higher and can contain metals, such as iron, zirconium, calcium, manganese, magnesium, and yttrium, alone or in combination of two or more.

Examples of the surfactant include a nonionic surfactant, an anionic surfactant, and a cationic surfactant, specifically, a polyoxyethylene/methylpolysiloxane copolymer, a poly(oxyethylene/oxypropylene) methylpolysiloxane copolymer, crosslinked polyether-modified silicone, crosslinked alkyl polyether-modified silicone, a cetyl dimethicone copolyol, propylene glycol monostearate, sorbitan monooleate, glyceryl stearate, polyoxyethylene hydrogenated castor oil, polyoxyethylene alkyl ether, sorbitan sesquioleate, polyoxyethylene sorbitan monostearate, and diglyceryl monooleate. These surfactants may be used singly or in combinations of two or more thereof.

The content of the surfactant in the film-forming composition of the present invention is preferably 0 mass% or more and 10 mass% or less, more preferably 0.01 mass% or more and 7.5 mass% or less, further more preferably 0.05 mass% or more and 5 mass% or less, and particularly preferably 0.1 mass% or more and 3 mass% or less.

Examples of the polyol in a liquid state at 20°C include alkylene glycols, such as ethylene glycol, propylene glycol, 1,3-propanediol, and 1,3-butanediol; polyalkylene glycols, such as diethylene glycol, dipropylene glycol, polyethylene glycol (preferably polyethylene glycol having a weight-average molecular weight of 2 000 or less), and polypropylene glycol; and glyceryls, such as glyceryl, diglyceryl, and triglyceryl. These may be used alone or in combination of two or more.

Among them, preferred are ethylene glycol, propylene glycol, 1,3-butanediol, dipropylene glycol, polyethylene glycol having a weight-average molecular weight of 2 000 or less, glyceryl, and diglyceryl, and more preferred are propylene glycol, 1,3-butanediol, polyethylene glycol having a weight-average molecular weight of 2 000 or less, and glyceryl.

Examples of the water-soluble polymer include a carbomer, an (acrylates/C10-30 alkyl acrylate) crosspolymer, a (Na acrylate/Na acryloyldimethyl taurate) copolymer, an (ammonium acryloyldimethyl taurate/beheneth-25 metacrylate) crosspolymer, an (ammonium acryloyldimethyl taurate/VP) copolymer, an (ammonium acryloyldimethyl taurate/carboxyethylammonium acrylate) crosspolymer, a (VA/crotonic acid) copolymer, an (acrylates/steareth-20 methacrylate) copolymer, an (acrylates/beheneth-25 metacrylate) copolymer, an acrylates copolymer, an (acrylates/vinyl neodecanoate) crosspolymer, a (PEG-150/decyl alcohol/SMDI) copolymer, and a (PEG-150/stearyl alcohol/SMDI) copolymer. These may be used alone or in combination of two or more.

The content of the water-soluble polymer in the film-forming composition of the present invention is preferably 0 mass% or more and 10 mass% or less, more preferably 0.001 mass% or more and 5 mass% or less, further more preferably 0.005 mass% or more and 3 mass% or less, and particularly preferably 0.01 mass% or more and 1 mass% or less.

Examples of the preservative include phenoxyethanol, methyl para-oxybenzoate, ethyl para-oxybenzoate, propyl para-oxybenzoate, isopropyl para-oxybenzoate, butyl para-oxybenzoate, isobutyl para-oxybenzoate, benzyl para-oxybenzoate, ethyl para-aminobenzoate, and ethylhexanediol. These may be used alone or in combination of two or more.

The film-forming composition of the present invention can be manufactured by a usual method, for example, by heating each component as needed and mixing them.

The film-forming composition of the present invention, when applied to skin and it is dried, forms a highly uniform film by spreading of the nonvolatile oil containing an ultraviolet absorber over the application area, has an excellent ultraviolet protection effect, and is unlikely to cause uneven suntan even if it is applied unevenly. In addition, the application procedure to skin is also simple, and it is easy to apply to a wide range.

The present inventors infer the reason why the effect of preventing uneven suntan is obtained even if the application is uneven is that when the film-forming composition of the present invention is applied to skin, the fiber of the component (b) adheres uniformly to the skin and forms network when dries, and the nonvolatile oil containing an ultraviolet absorber further spreads through the fiber of the component (b) to form a highly uniform film.

Whether the fiber forms network in the film or not can be verified by a scanning electron microscope (SEM). The network is a state that fibers dispersed in the film have intersections with each other to have gaps between the fibers and is a state that the component contained in the film-forming composition is retained in the gaps. The intersection of fibers is preferably a state that, for example, one fiber has two or more intersections with other two or more fibers, and the fibers are involved with each other.

The form of the film-forming composition of the present invention is not particularly limited and may be a liquid, a solid, or a semisolid. The form may be, for example, an emulsion, paste, a cream, a gel, a lotion, a transparent liquid, a spray (aerosol type or non-aerosol type), a foam (aerosol type or non-aerosol type), a solid formulation, or a semisolid formulation or may be a sheet agent in which a sheet base is impregnated with the film-forming composition of the present invention as an impregnating liquid.

Examples of the film-forming composition of the present invention include an emulsified composition (specifically, an oil-in-water type emulsified composition and a water-in-oil type emulsified composition), a multi-layered composition, and an oil composition.

The film-forming composition of the present invention is suitable for use as a cosmetic, in particular, as sunscreen. The film-forming composition of the present invention is suitable for use as a skin cosmetic, more suitable for application to the skin excluding the scalp, and further suitable for application to one or more selected from the group consisting of the face, the body, and the limbs.

Examples of means for applying the film-forming composition of the present invention to the skin include a finger application, a spray application, an application using a tool such as a roller or sponge, and an application with a solid stick-shaped cosmetic.

The thickness of thus-formed film of the present invention varies in accordance with the applied amount and is, in normal use (application basis weight: 1 mg/cm$^2$ or more and 3 mg/cm$^2$ or less), preferably 0.3 $\mu$m or more and 30 $\mu$m or less and more preferably 0.5 $\mu$m or more and 20 $\mu$m or less. The thickness of a film may be measured with a contact coating thickness gauge (manufactured by Mitutoyo Corporation, Litematic VL-50A) after application of the film-forming composition to a PET substrate.

Regarding the above-described embodiments, the present invention further discloses the following film-forming composition and the like.

<1> A film-forming composition comprising components (a) and (b):

(a) a nonvolatile oil containing an ultraviolet absorber; and

(b) a fiber having an average fiber diameter of 0.1 $\mu$m or more and 7 $\mu$m or less in an amount of 0.5 mass% or more and 10 mass% or less in the film-forming composition, wherein

(average fiber diameter)$^2$/(fiber content) ($\mu$m$^2$/mass%) is 0.005 or more and 7 or less.

<2> The film-forming composition according to <1>, wherein the component (a) is preferably a nonvolatile liquid oil containing an ultraviolet absorber.

<3> The film-forming composition according to <1> or <2>, wherein the content of the component (a) in the film-forming composition is preferably 0.5 mass% or more, more preferably 2.5 mass% or more, further more preferably 5 mass% or more, further more preferably 7.5 mass% or more, and particularly preferably 10 mass% or more, and the content in the film-forming composition is preferably 90 mass% or less, more preferably 65 mass% or less, further more preferably 45 mass% or less, and particularly preferably 40 mass% or less.

<4> The film-forming composition according to <1> or <2>, wherein the content of the component (a) in the film-forming composition is preferably 0.5 mass% or more and 90 mass% or less, more preferably 2.5 mass% or more and 65 mass% or less, further more preferably 5 mass% or more and 45 mass% or less, further more preferably 7.5 mass% or more and 45 mass% or less, and particularly preferably 10 mass% or more and 40 mass% or less.

<5> The film-forming composition according to any one of <1> to <4>, wherein the content of the ultraviolet absorber in the film-forming composition is preferably 0.1 mass% or more, more preferably 1 mass% or more, further more preferably 3 mass% or more, and particularly preferably 7 mass% or more, and the content in the film-forming composition is preferably 55 mass% or less, more preferably 50 mass% or less, further more preferably 45 mass% or less, and particularly preferably 40 mass% or less.

<6> The film-forming composition according to any one of <1> to <4>, wherein the content of the ultraviolet absorber in the film-forming composition is preferably 0.1 mass% or more and 55 mass% or less, more preferably 1 mass% or more and 50 mass% or less, further more preferably 3 mass% or more and 45 mass% or less, and particularly preferably 7 mass% or more and 40 mass% or less.

<7> The film-forming composition according to any one of <1> to <6>, wherein a mass ratio of the ultraviolet absorber to a total of the nonvolatile oil is preferably 0.1 or more, more preferably 0.3 or more, further more preferably 0.5 or more, and particularly preferably 0.7 or more and is preferably 1 or less.

<8> The film-forming composition according to any one of <1> to <6>, wherein the mass ratio of the ultraviolet absorber to the total of the nonvolatile oil is preferably 0.1 or more and 1 or less, more preferably 0.3 or more and 1 or less, further more preferably 0.5 or more and 1 or less, and particularly preferably 0.7 or more and 1 or less.

<9> The film-forming composition according to any one of <1> to <8>, wherein the ultraviolet absorber is preferably one or more selected from the group consisting of benzoic acid-based ultraviolet absorbers, anthranilic acid-based ultraviolet absorbers, salicylic acid-based ultraviolet absorbers, cinnamic acid-based ultraviolet absorbers, benzoylmethane-based ultraviolet absorbers, triazine-based ultraviolet absorbers, benzophenone-based ultraviolet absorbers, and hydantoin-based ultraviolet absorbers, and more preferably one or more selected from the group consisting of benzoic acid-based ultraviolet absorbers, cinnamic acid-based ultraviolet absorbers, and triazine-based ultraviolet absorbers, and particularly preferably one or more selected from the group consisting of para-aminobenzoic acid, glyceryl para-aminobenzoate, ethyldihydroxypropyl para-aminobenzoate, octyldimethyl para-aminobenzoate, amyl para-dimethylaminobenzoate, diethylamino hydroxybenzoyl hexyl benzoate, 2-ethylhexyl para-methoxycinnamate, glyceryl mono-2-ethylhexanoate di-para-methoxycinnamate, methyl 2,5-diisopropylcinnamate, methylbis (trimethylsiloxy)silylisopentyl trimethoxycinnamate, isopropyl para-methoxycinnamate, a mixture of isopropyl para-methoxycinnamate and diisopropyl cinnamate, 2-ethoxyethyl para-methoxycinnamate, diethanolamine para-methoxycinnamate, 2,4,6-tris[4-(2-ethylhexyloxycarbonyl)anilino]-1,3,5-triazine, and 2,4-bis-[{4-(2-ethylhexyloxy)-2-hydroxy}-phenyl]-6-(4-methoxyphenyl)-1,3,5-triazine.

<10> The film-forming composition according to any one of <1> to <9>, further containing a nonvolatile oil other than an oil-soluble ultraviolet absorber.

<11> The film-forming composition according to <10>, wherein the nonvolatile oil other than an oil-soluble ultraviolet absorber is preferably one or more oils selected from the group consisting of ester oils, ether oils, hydrocarbon oils, higher alcohols, silicone oils, and fluorine oils, more preferably one or more oils selected from the group consisting of ester oils, ether oils, hydrocarbon oils, and higher alcohols, and particularly preferably one or more oils selected from the group consisting of ester oils, ether oils, and hydrocarbon oils.

<12> The film-forming composition according to <11>, wherein the ester oil is preferably one or more selected from the group consisting of octyldodecyl myristate, myristyl myristate, isocetyl stearate, isocetyl isostearate, cetearyl isononanoate, diisobutyl adipate, di-2-ethylhexyl sebacate, isopropyl myristate, isopropyl palmitate, diisostearyl malate, neopentyl glycol dicaprate, C12-15 alkyl benzoate, and glyceryl tri(caprylate/caprate), more preferably one or more selected from the group consisting of isopropyl myristate, isopropyl palmitate, diisostearyl malate, neopentyl glycol dicaprate, C12-15 alkyl benzoate, and glyceryl tri(caprylate/caprate), further more preferably one or more selected from the group consisting of isopropyl myristate, neopentyl glycol dicaprate, C12-15 alkyl benzoate, and glyceryl tri(caprylate/caprate), and particularly preferably one or two selected from the group consisting of neopentyl glycol dicaprate and C12-15 alkyl benzoate.

<13> The film-forming composition according to <10>, wherein the nonvolatile oil other than an oil-soluble ultraviolet absorber is preferably a nonvolatile oil having an IOB value of exceeding 0 and 0.6 or less and more preferably a nonvolatile oil having an IOB value of 0.05 or more and 0.3 or less.

<14> The film-forming composition according to any one of <1> to <13>, wherein the component (b) preferably has an average fiber diameter of 0.2 $\mu$m or more, more preferably 0.3 $\mu$m or more, and further more preferably 0.4 $\mu$m or more and preferably 5 $\mu$m or less, more preferably 4 $\mu$m or less, further more preferably 3 $\mu$m or less, further preferably 1.5 $\mu$m or less, and particularly preferably 0.8 $\mu$m or less.

<15> The film-forming composition according to any one of <1> to <13>, wherein the component (b) preferably has an average fiber diameter of 0.2 $\mu$m or more and 5 $\mu$m or less, more preferably 0.3 $\mu$m or more and 4 $\mu$m or less, further more preferably 0.4 $\mu$m or more and 3 $\mu$m or less, further more preferably 0.4 $\mu$m or more and 1.5 $\mu$m or less, and particularly preferably 0.4 $\mu$m or more and 0.8 $\mu$m or less.

<16> The film-forming composition according to any one of <1> to <15>, wherein the component (b) preferably has an average fiber length of 1 $\mu$m or more, more preferably 3 $\mu$m or more, further more preferably 20 $\mu$m or more, further more preferably 25 $\mu$m or more, further more preferably 30 $\mu$m or more, and particularly preferably 40 $\mu$m or more and preferably 300 $\mu$m or less, more preferably 250 $\mu$m or less, further more preferably 200 $\mu$m or less, and particularly preferably 150 $\mu$m or less.

<17> The film-forming composition according to any one of <1> to <15>, wherein the component (b) preferably has an average fiber length of 1 $\mu$m or more and 300 $\mu$m or less, more preferably 3 $\mu$m or more and 300 $\mu$m or less, further more preferably 20 $\mu$m or more and 300 $\mu$m or less, further more preferably 25 $\mu$m or more and 250 $\mu$m or less, further more preferably 30 $\mu$m or more and 200 $\mu$m or less, and particularly preferably 40 $\mu$m or more and 150 $\mu$m or less.

<18> The film-forming composition according to any one of <1> to <17>, wherein the fiber of the component (b) preferably has an aspect ratio, (average fiber length/average fiber diameter), of 8 or more, more preferably 10 or more, further more preferably 15 or more, further more preferably 20 or more, further more preferably 30 or more, and particularly preferably 60 or more and preferably 300 or less, more preferably 250 or less, further more preferably 200 or less, and particularly preferably 150 or less.

<19> The film-forming composition according to any one of <1> to <18>, wherein the component (b) is preferably a fiber of a water-insoluble polymer.

<20> The film-forming composition according to <19>, wherein the water-insoluble polymer is preferably one or more selected from the group consisting of polyvinyl alcohols such as a completely saponified polyvinyl alcohol that can be insolubilized after film formation and a partially saponified polyvinyl alcohol that can be crosslinked after film formation by using a crosslinking agent together, a poly(N-propanoylethyleneimine) graft-dimethylsiloxane/y-aminopropylmethylsiloxane copolymer, polyvinylacetal diethylaminoacetate, Zein (major component of corn protein), polylactic acid (PLA), polybutylene succinate, polyglycolic acid, polycaprolactone, polyhydroxy alkanoic acid, polylactic acid (PLA), polyethylene terephthalate (PET), polybutylene terephthalate, a polymer containing one or more structural units selected from the group consisting of a structural unit derived from (meth)acrylic acid or a salt thereof, a structural unit derived from (meth)acrylic acid ester, a structural unit derived from (meth)acrylonitrile, a structural unit derived from (meth)acrylamide, and a structural unit derived from N-substituted (meth)acrylamide, cellulose, chitin, chitosan, Nylon, a polyimide resin, a polyamideimide resin, a polystyrene resin, a polyvinylbutyral resin, a polyvinylacetal resin, a polyurethane resin, a polypropylene resin, a polyethylene resin, and various polypeptides.

<21> The film-forming composition according to <19>, wherein the water-insoluble polymer is preferably one or more selected from the group consisting of oxazoline-modified silicone, a biodegradable resin (more preferably polyvinylacetal diethylaminoacetate, Zein, or polylactic acid), a polyester resin, a (meth)acrylic resin, a water-insoluble polysaccharide, a polyamide resin, a completely saponified polyvinyl alcohol that can be insolubilized after film formation, a partially saponified polyvinyl alcohol that can be crosslinked after film formation by using a crosslinking agent together, a polyvinylbutyral resin, a polyurethane resin, and a polypropylene resin, more preferably one or more selected from the group consisting of a polyester resin, a (meth)acrylic resin, a water-insoluble polysaccharide, a polyamide resin, a polyvinylbutyral resin, a polyurethane resin, and a polypropylene resin, further more preferably one or more selected from the group consisting of a polyester resin, a (meth)acrylic resin, a water-insoluble polysaccharide, and a polyamide resin, further more preferably one or more selected from a group consisting of a polyester resin and a (meth)acrylic resin, and particularly preferably a (meth)acrylic resin.

<22> The film-forming composition according to <21>, wherein the (meth)acrylic resin is preferably an (alkyl (meth)acrylamide/hydroxyalkyl (meth)acrylate/alkylaminoalkyl (meth)acrylate) copolymer and more preferably an (octyl (meth)acrylamide/hydroxypropyl (meth)acrylate/butylaminoethyl (meth)acrylate) copolymer.

<23> The film-forming composition according to any one of <1> to <22>, wherein the content of the fiber of the component (b) in the film-forming composition is preferably 0.7 mass% or more, more preferably 1 mass% or more, further more preferably 2 mass% or more, and particularly preferably 3 mass% or more, and the content in the film-forming composition is preferably 9 mass% or less, more preferably 8.5 mass% or less, further more preferably 8 mass% or less, further more preferably 6 mass% or less, and particularly preferably 5 mass% or less.

<24> The film-forming composition according to any one of <1> to <22>, wherein the content of the fiber of the component (b) in the film-forming composition is preferably 0.7 mass% or more and 9 mass% or less, more preferably 1 mass% or more and 8.5 mass% or less, further more preferably 2 mass% or more and 8 mass% or less, further more preferably 3 mass% or more and 6 mass% or less, and particularly preferably 3 mass% or more and 5 mass% or less.

<25> The film-forming composition according to any one of <1> to <24>, wherein the mass ratio of the component (b) to the component (a), (b)/(a), is preferably 0.005 or more, more preferably 0.02 or more, further more preferably 0.05 or more, and particularly preferably 0.1 or more and is preferably 5 or less, more preferably 4 or less, further more preferably 3 or less, further more preferably 2 or less, further more preferably 0.4 or less, further more preferably 0.25 or less, and particularly preferably 0.2 or less.

<26> The film-forming composition according to any one of <1> to <24>, wherein the mass ratio of the component (b) to the component (a), (b)/(a), is preferably 0.005 or more and 5 or less, more preferably 0.02 or more and 4 or less, further more preferably 0.05 or more and 3 or less, further more preferably 0.1 or more and 2 or less, further more preferably 0.1 or more and 0.4 or less, further more preferably 0.1 or more and 0.25 or less, and particularly preferably 0.1 or more and 0.2 or less.

<27> The film-forming composition according to any one of <1> to <26>, wherein the (average fiber diameter)$^2$/(fiber content) ($\mu$m$^2$/mass%) of the component (b) is preferably 0.01 or more, more preferably 0.02 or more, further more preferably 0.03 or more, and particularly preferably 0.05 or more and is preferably 6 or less, more preferably 5 or less, further more preferably 4 or less, further more preferably 3 or less, further more preferably 1 or less, further more preferably 0.5 or less, further more preferably 0.2 or less, and particularly preferably 0.1 or less.

<28> The film-forming composition according to any one of <1> to <26>, wherein the (average fiber diameter)$^2$/(fiber content) ($\mu$m$^2$/mass%) of the component (b) is preferably 0.01 or more and 6 or less, more preferably 0.02 or more and 5 or less, further more preferably 0.03 or more and 4 or less, further more preferably 0.05 or more and 3 or less, further more preferably 0.05 or more and 1 or less, further more preferably 0.05 or more and 0.5 or less, further more preferably 0.05 or more and 0.2 or less, and particularly preferably 0.05 or more and 0.1 or less.

<29> The film-forming composition according to any one of <1> to <28>, wherein the (average fiber diameter)$^2$/(fiber content) ($\mu$m$^2$/mass%) of the component (b) is 0.02 or more and 0.5 or less, the fiber of the component (b) has an aspect ratio, (average fiber length/average fiber diameter), of 15 or more, and the content of the ultraviolet absorber in the film-forming composition is 7 mass% or more.

<30> The film-forming composition according to any one of <1> to <29>, wherein the (average fiber diameter)$^2$/(fiber content) ($\mu$m$^2$/mass%) of the component (b) is 0.02 or more and 0.5 or less, the fiber of the component (b) has an aspect ratio, (average fiber length)/(average fiber diameter), of 15 or more, and the mass ratio of the ultraviolet absorber to the total of the nonvolatile oil is 0.5 or more.

<31> The film-forming composition according to any one of <1> to <30>, further containing a volatile component.

<32> The film-forming composition according to <31>, wherein the volatile component is preferably one or more selected from the group consisting of water, alcohol, amides, ketone, and volatile silicone oil and more preferably one or more selected from the group consisting of water, alcohol and volatile silicone oil.

<33> The film-forming composition according to <31> or <32>, wherein the content of the volatile component in the film-forming composition is preferably 20 mass% or more, more preferably 30 mass% or more, further more preferably 45 mass% or more, and particularly preferably 50 mass% or more, and the content in the film-forming composition is preferably 99 mass% or less, more preferably 95 mass% or less, further more preferably 90 mass% or less, and particularly preferably 85 mass% or less.

<34> The film-forming composition according to any one of <1> to <33>, further containing one or more selected from the group consisting of a powder, a surfactant, an emulsion stabilizer, a polyol in a liquid state at 20°C, a water-soluble polymer, a preservative, a pH adjuster, a moisturizing agent, an algefacient, an amino acid, a perfume, and a metal sequestering agent.

<35> The film-forming composition according to any one of <1> to <34>, wherein the composition is preferably a liquid, a solid, or a semisolid and more preferably an emulsion, a paste, a cream, a gel, a lotion, a transparent liquid, a spray (aerosol type or non-aerosol type), a foam (aerosol type or non-aerosol type), a solid formulation, or a semisolid formulation.

<36> The film-forming composition according to any one of <1> to <35>, wherein the composition is preferably a skin cosmetic, more preferably a cosmetic to be used by application to the skin excluding the scalp, and further more preferably a cosmetic to be used by application to one or more selected from the group consisting of the face, the body, and the limbs.

<37> The film-forming composition according to any one of <1> to <35>, wherein the composition is preferably a cosmetic and more preferably sunscreen.

<38> A method for manufacturing a film on a skin surface, comprising applying the film-forming composition according to any one of <1> to <37> to skin.

<39> A film including the film-forming composition according to any one of <1> to <37>.

<40> The film according to <39>, wherein the thickness of the film when the application basis weight is 1 $mg/cm^2$ or more and 3 $mg/cm^2$ or less is preferably 0.3 $\mu$m or more and 30 $\mu$m or less and more preferably 0.5 $\mu$m or more and 20 $\mu$m or less.

Examples

The present invention will now be described in detail with reference to examples, but is not limited to the examples. The methods for measuring the average fiber diameter and average fiber length of a fiber are as follows.

(Method for measuring average fiber diameter)

The average fiber diameter was determined by observing fibers with an SEM at a magnification of 2 000 times or 5 000 times, arbitrarily selecting 100 fibers excluding defects (e.g., clumps of fibers and crossing parts of fibers) from the two-dimensional image, drawing a line perpendicular to the longitudinal direction of each of the fibers to directly read the fiber diameters as measured values, and arithmetically averaging them to determine the average fiber diameter.

(Method for measuring average fiber length)

The average fiber length was determined by observing fibers with an SEM at a magnification of 250 times to 750 times according to the lengths of the fibers, arbitrarily selecting 100 fibers excluding defects (e.g., clumps of fibers and crossing parts of fibers) from the two-dimensional image, drawing a line in the longitudinal direction of each of the fibers to directly read the fiber lengths as measured values, and arithmetically averaging them to determine the average fiber length.

(Method for measuring anionic group content of anion-modified cellulose fiber)

A dispersion was prepared by placing 0.5 g (dry mass) of a cellulose fiber as a measurement object in a beaker, adding deionized water or a solvent mixture of methanol/water = 2/1 (volume ratio) thereto to a total volume of 55 mL, and adding 5 mL of a 0.01 M sodium chloride aqueous solution thereto. The dispersion was thoroughly stirred until the cellulose fiber is sufficiently dispersed. The pH of this dispersion was adjusted to 2.5 to 3 with 0.1 M hydrochloric acid, and a 0.05 M sodium hydroxide aqueous solution was then dropwise added to the dispersion with a waiting time of 60 seconds by using an automatic titrator (manufactured by DKK-TOA Corporation, trade name: "AUT-701"), and conductivity and pH value were measured every minute. The measurement was continued until the pH reached about 11 to obtain a conductivity curve. The sodium hydroxide titer was determined from this conductivity curve, and the anionic group content of the cellulose fiber as the measuring object was calculated by the following expression:

$$\text{Anionic group content (mmol/g)} = \text{[sodium hydroxide aqueous solution titer (mL)} \times \text{sodium hydroxide aqueous solution concentration (0.05 M)]/[mass (0.5 g) of cellulose fiber as measuring object].}$$

(Method for measuring metal ion amount bound to anionic group)

A mixture of about 1 g of a dried anion-modified cellulose fiber and 100 g of deionized water were stirred (100 rpm) at 20°C for 1 hour, followed by filtration using a filter paper (No. 5). This washing procedure was performed three times in total. The anion-modified cellulose fiber after the washing was lyophilized, weighed, ashed, and dissolved in hydrochloric acid, and the metal ion amount was measured by using an ICP emission spectrophotometer (manufactured by Thermo Fisher Scientific, iCAP 6500Duo) by inductive coupled plasma-atomic emission spectrometry (ICP-AES).

[Manufacturing example A1: manufacture of short fiber (component (b))]

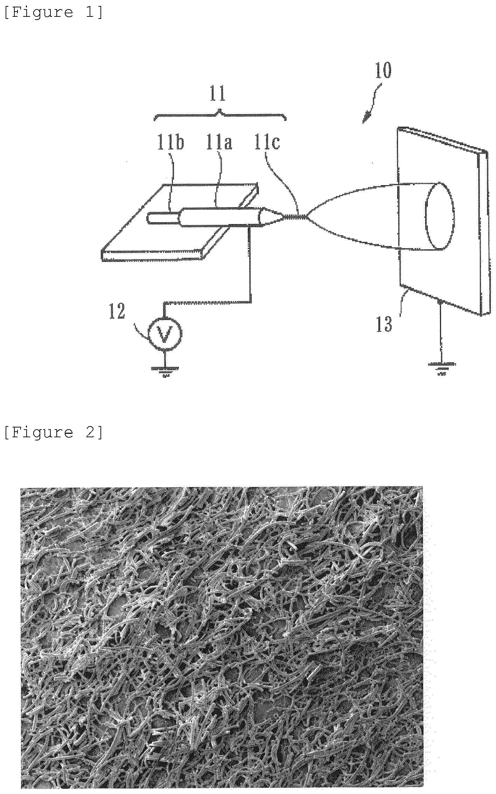

(1) The acrylic resin ((octyl acrylamide/hydroxypropyl acrylate/butylaminoethyl methacrylate) copolymer) in Table 2 was dissolved in ethanol to obtain an 18 mass% solution. A nanofiber sheet was formed on the surface of a collector using this solution with the device shown in Figure 1 by an electrospinning method. The conditions for manufacturing the nanofiber are as follows:

Applied voltage: 30 kV;
Capillary-collector distance: 150 mm;
Solution discharge amount: 12 mL/hour; and
Environment: 25°C, 30%RH

(2) The obtained nanofiber sheet was cut into an appropriate size and was then pulverized with an agitation system (manufactured by PRIMIX Corporation, LABOLUTION (registered trademark)) attached with dispersal blades at a rotational speed of 5 000 rpm for 30 minutes to obtain fibers.

[Manufacturing examples A2 and A3: manufacture of short fiber (component (b))]

Fibers were manufactured as in manufacturing example A1 except that the polymer concentration, rotational speed, shearing time were those shown in Table 1.

[Manufacturing example B1: manufacture of short fiber]

A fiber was manufactured as in manufacturing example A1 except that the polymer concentration, rotational speed, shearing time were those shown in Table 1.

[Table 1]

| | Manufacturing example A2 | Manufacturing example A3 | Manufacturing example B1 |
|---|---|---|---|
| Polymer concentration (mass%) | 15 | 24 | 28 |
| Rotational speed (rpm) | 4000 | 7000 | 8000 |
| Shearing time (min) | 30 | 30 | 30 |

[Manufacturing example A4: manufacture of short fiber (component (b))]

(1) The ester resin (polylactic acid) shown in Table 2 was dissolved in a mixture of chloroform and dimethylformamide (mass ratio = 80 : 20) to obtain a 10 mass% solution. A nanofiber sheet was formed on the surface of a collector using this solution with the device shown in Figure 1 by an electrospinning method. The conditions for manufacturing the nanofiber are as follows:

Applied voltage: 30 kV;
Capillary-collector distance: 150 mm;
Solution discharge amount: 12 mL/hour; and
Environment: 25°C, 30%RH.

(2) The nanofiber sheet obtained was charged in a dispersion device (manufactured by Taiheiyo Kiko K.K., Milder) and was sheared at a rotational speed of 13 500 rpm and a circulation number of 8 in a circulation line to obtain a fiber.

[Manufacturing example B2: manufacture of short fiber]

A fiber was manufactured as in manufacturing example A4 except that the polymer concentration was 20 mass%, the rotational speed was 13 500 rpm, and the circulation number was 16.

[Examples 1 to 12 and Comparative Examples 1 to 4: oil-in-water type emulsified composition]

(Manufacturing method)

Oil-in-water type emulsified compositions were formulated by using the fibers obtained in manufacturing examples A1 to A4, B1, and B2 according to the prescriptions shown in Tables 2 to 4. The characteristics of the obtained fibers are shown in Tables 2 to 4.

(Measurement method and evaluation method)

(1) Network formability

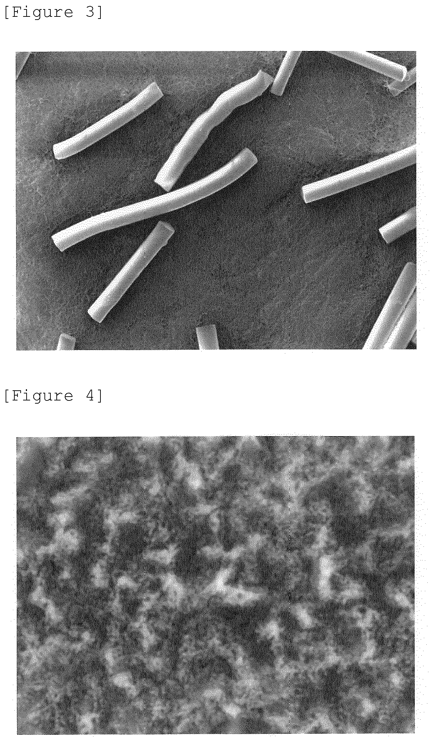

The oil-in-water type emulsified compositions obtained above were applied to artificial leather at 2 mg/cm$^2$ to form films. The formed films were each evaluated for whether a network was formed or not with an SEM (manufactured by JEOL Ltd., JSM-IT500, magnification: 500 times). In the evaluation of network formation, when a fiber had two or more intersections with other fibers to show a state in which gaps surrounded by fibers existed as a whole, it was determined that a network was formed, and it was expressed as "Presence" in the tables. When a state in which gaps surrounded by fibers existed was almost not observed, it was determined that network was not formed, and it was expressed as "Absence" in the tables.
The results are shown in Tables 2 to 4. Figures 2 and 3 show a reference SEM image in the presence of network formation and a reference SEM image in the absence of network formation, respectively, used as standards for determining whether a network was formed or not in Examples and Comparative Example.

(2) Film uniformity

The compositions obtained above were applied to a PMMA plate (manufactured by Helioscreen, HELIOPLATE HD) at 2 mg/cm$^2$ to form films. After 15 minutes from the application, the CV value in the absorbance of the ultraviolet (310 nm) film image was measured. The uniformity of each film was evaluated from this CV value in four grades in accordance with the following criterion. The results are shown in Tables 2 to 4.

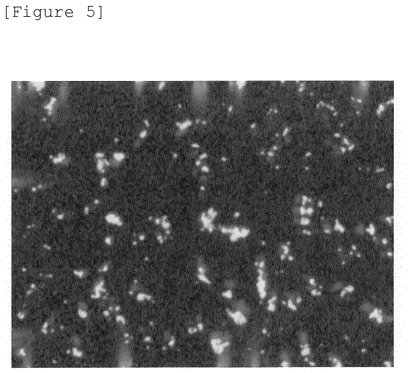

Figure 4 shows the state of the film immediately after the application of the oil-in-water type emulsified composition of Example 2, and Figure 5 shows the state of the film after 15 minutes from the application. As shown in Figures 4 and 5, in the oil-in-water type emulsified composition of Example 2, the nonvolatile oil containing an ultraviolet absorber spread over the application area over time to increase the uniformity.

(Evaluation criterion for film uniformity)

4: CV value of 0% or more and less than 20%
3: CV value of 20% or more and less than 30%
2: CV value of 30% or more and less than 40%
1: CV value of 40% or more

(3) Ultraviolet protection effect (in vitro SPF)

The compositions obtained above were applied to a PMMA plate (manufactured by Helioscreen, HELIOPLATE HD) at 2 mg/cm$^2$ to form films. After 15 minutes from the application, the in vitro SPF was evaluated with an SPF analyzer (manufactured by Labsphere, Inc., UV-2000S). The results are shown in Tables 2 to 4.

[Table 2]

| Component (mass%) | | | Example | | | | | | | | |
|---|---|---|---|---|---|---|---|---|---|---|---|
| | | | 1 | 2 | 3 | 4 | 5 | 6 | 7 | 8 | 9 |
| Fiber | | Manufacturing example No. | A1 | A1 | A2 | A3 | A2 | A3 | A1 | A1 | A4 |
| | | Polymer type | Acrylic resin (*1) | Acrylic resin (*1) | Acrylic resin (*1) | Acrylic resin (*1) | Acrylic resin (*1) | Acrylic resin (*1) | Acrylic resin (*1) | Acrylic resin (*1) | Polylacti c acid (*2) |
| | | Average fiber diameter X ($\mu$m) | 0.5 | 0.5 | 0.3 | 2 | 0.3 | 2 | 0.5 | 0.5 | 0.7 |
| | | Average fiber length Y ($\mu$m) | 50 | 50 | 50 | 50 | 50 | 50 | 50 | 50 | 50 |
| | | Aspect ratio Y/X | 100 | 100 | 166.7 | 25 | 166.7 | 25 | 100 | 100 | 71.4 |
| | | (Average fiber diameter)$^2$/(fiber content)($\mu$m$^2$/mass%) (cumulative fiber length) | 0.0625 | 0.0625 | 0.0225 | 1 | 0.0150 | 2 | 0.125 | 0.0313 | 0.123 |
| | | Fiber content (mass%) | 4 | 4 | 4 | 4 | 6 | 2 | 2 | 8 | 4 |
| Component (a) | UV absorber | Ethylhexyl methoxycinnamate (*3) | 10 | 16 | 16 | 16 | 16 | 16 | 16 | 16 | 16 |
| | | Diethylaminohydroxybe nzoyl hexyl benzoate (*4) | 2 | - | - | - | - | - | - | - | - |
| | | Bis-ethylhexyloxyphenol methoxyphenyl triazine (*5) | 2 | - | - | - | - | - | - | - | - |
| | | Ethylhexyl triazone (*6) | 2 | - | - | - | - | - | - | - | - |
| | | C12-15 alkyl benzoate (*7) | 2 | 2 | 2 | 2 | 2 | 2 | 2 | 2 | 2 |
| Water | | | 76.91 | 76.91 | 76.91 | 76.91 | 74.91 | 78.91 | 78.91 | 72.91 | 76.91 |
| (Acrylates/C10-30 alkyl acrylate) crosspolymer (*8) | | | 0.15 | 0.15 | 0.15 | 0.15 | 0.15 | 0.15 | 0.15 | 0.15 | 0.15 |
| Isoceteth-20 | | | 0.5 | 0.5 | 0.5 | 0.5 | 0.5 | 0.5 | 0.5 | 0.5 | 0.5 |

(continued)

| Component (mass%) | | Example | | | | | | | | |
|---|---|---|---|---|---|---|---|---|---|---|
| | | 1 | 2 | 3 | 4 | 5 | 6 | 7 | 8 | 9 |
| Preservative | | 0.34 | 0.34 | 0.34 | 0.34 | 0.34 | 0.34 | 0.34 | 0.34 | 0.34 |
| pH adjuster | | 0.1 | 0.1 | 0.1 | 0.1 | 0.1 | 0.1 | 0.1 | 0.1 | 0.1 |
| Total | | 100 | 100 | 100 | 100 | 100 | 100 | 100 | 100 | 100 |
| Evaluation | Network formability | Presence | Presence | Presence | Presence | Presence | Presence | Presence | Presence | Presenc e |
| | Film uniformity | 4 | 4 | 4 | 3 | 3 | 3 | 4 | 4 | 4 |
| | UV protection effect | 20.50 | 9.44 | 8.60 | 7.07 | 9.22 | 6.56 | 10.44 | 8.46 | 7.80 |

[Table 3]

| Component (mass%) | | | Example | | |
|---|---|---|---|---|---|
| | | | 10 | 11 | 12 |
| Fiber | | Manufacturing example No. | A1 | A1 | A1 |
| | | Polymer type | Acrylic resin (*1) | Acrylic resin (*1) | Acrylic resin (*1) |
| | | Average fiber diameter X ($\mu$m) | 0.5 | 0.5 | 0.5 |
| | | Average fiber length Y ($\mu$m) | 50 | 50 | 50 |
| | | Aspect ratio Y/X | 100 | 100 | 100 |
| | | (Average fiber diameter)$^2$/(fiber content)($\mu$m$^2$/mass%) (cumulative fiber length) | 0.0625 | 0.0625 | 0.0625 |
| | | Fiber content(mass%) | 4 | 4 | 4 |
| Component (a) | UV absorber | Ethylhexyl methoxycinnamate (*3) | 6.25 | 3.13 | 40 |
| | | Diethylaminohydroxybenzoyl hexyl benzoate (*4) | 1.25 | 0.63 | - |
| | | Bis-ethylhexyloxyphenol methoxyphenyl triazine (*5) | 1.25 | 0.63 | - |
| | | Ethylhexyl triazone (*6) | 1.25 | 0.63 | - |
| | | C12-15 alkyl benzoate (*7) | 5 | 8 | - |
| Water | | | 79.91 | 81.91 | 54.91 |
| (Acrylates/C10-30 alkyl acrylate) crosspolymer (*8) | | | 0.15 | 0.15 | 0.15 |
| Isoceteth-20 | | | 0.5 | 0.5 | 0.5 |
| Preservative | | | 0.34 | 0.34 | 0.34 |
| pH adjuster | | | 0.1 | 0.1 | 0.1 |
| (Total | | | 100 | 100 | 100 |
| Evaluation | | Network formability | Presence | Presence | Presence |
| | | Film uniformity | 4 | 3 | 4 |
| | | UV protection effect | 16.20 | 8.23 | 24.65 |

[Table 4]

| Component (mass%) | | | Comparative Example | | | |
|---|---|---|---|---|---|---|
| | | | 1 | 2 | 3 | 4 |
| Fiber | | Manufacturing example No. | B2 | B1 | A1 | A2 |
| | | Polymer type | Polylactic acid (*2) | Acrylic resin (*1) | Acrylic resin (*1) | Acrylic resin (*1) |
| | | Average fiber diameter X ($\mu$m) | 10 | 5 | 0.5 | 0.3 |
| | | Average fiber length Y ($\mu$m) | 50 | 50 | 50 | 50 |
| | | Aspect ratio Y/X | 5 | 10 | 100 | 166.7 |
| | | (Average fiber diameter)$^2$/(fiber content)($\mu$m$^2$/mass%) (cumulative fiber length) | 10 | 12.5 | 2.5 | 0.0075 |
| | | Fiber content(mass%) | 10 | 2 | 0.1 | 12 |
| Component (a) | UV absorber | Ethylhexyl methoxycinnamate (*3) | 16 | 16 | 16 | 16 |
| | | Diethylaminohydroxybenzoyl hexyl benzoate (*4) | - | - | - | - |
| | | Bis-ethylhexyloxyphenol methoxyphenyl triazine (*5) | - | - | - | - |
| | | Ethylhexyl triazone (*6) | - | - | - | - |
| | | C12-15 alkyl benzoate (*7) | 2 | 2 | 2 | 2 |
| Water | | | 70.91 | 78.91 | 80.81 | 68.91 |
| (Acrylates/C10-30 alkyl acrylate) crosspolymer (*8) | | | 0.15 | 0.15 | 0.15 | 0.15 |
| Isoceteth-20 | | | 0.5 | 0.5 | 0.5 | 0.5 |
| Preservative | | | 0.34 | 0.34 | 0.34 | 0.34 |
| pH adjuster | | | 0.1 | 0.1 | 0.1 | 0.1 |
| Total | | | 100 | 100 | 100 | 100 |
| | | | | | | |
| Evaluation | | Network formability | Absence | Absence | Absence | Presence |
| | | Film uniformity | 1 | 1 | 1 | 1 |
| | | UV protection effect | 3.60 | 5.87 | 5.32 | 5.05 |

The symbols in the tables show the followings:

*1: AMPHOMER 28-4910 (manufactured by Akzo Nobel N.V.);
*2: Ingeo 6252D (manufactured by NatureWorks LLC);
*3: Uvinul MC80 (manufactured by BASF SE);
*4: Uvinul A Plus (manufactured by BASF SE);
*5: TINOSORB S (manufactured by BASF SE);
*6: Uvinul T-150 (manufactured by BASF SE);
*7: Finsolv TN-O (manufactured by Innospec Performance Chemicals); and
*8: PEMULEN TR-1 (manufactured by Lubrizol Advanced Materials, Inc.).

[Manufacturing example A5: manufacture of short fiber (component (b))]

Ten grams of conifer bleached kraft pulp (manufactured by West Fraser Timber Co., Ltd. trade name: Hinton) as natural cellulose was thoroughly stirred in 990 g of ion exchanged water, and 0.13 g of 2,2,6,6-tetramethylpiperidine-1-oxyl (manufactured by Sigma-Aldrich Co. LLC, 98 mass%), 1.3 g of sodium bromide, and 27 g of a sodium hypochlorite aqueous solution (10.5 mass% aqueous solution) were added in this order to 10 g of the pulp. After completion of the reaction, washing and pH adjustment were performed.

On this occasion, by using pH-stat titration with an automatic titrator (manufactured by DKK-TOA Corporation, AUT-701), the pH was maintained at 10.5 by adding a 0.5 M sodium hydroxide aqueous solutiondropwise. After reaction was performed at 20°C and an agitation speed of 200 rpm for 120 minutes, thorough washing with ion exchanged water and then dehydration treatment were performed to obtain an anion-modified cellulose fiber with a solid content of 44.6%. The anionic group of the obtained anion-modified cellulose fiber was a carboxy group, and the carboxy group content was 1.0 mmol/g.

A mixture obtained by mixing 10.0 g (absolute dry mass) of the anion-modified cellulose fiber obtained above and 987.6 g of ion exchanged water was subjected to refinement by using a high-pressure homogenizer (manufactured by Yoshida Kikai Co., Ltd., Nanoveita L-ES) to obtain a fine cellulose fiber dispersion containing a carboxy group as the anionic group. The solid concentration of the obtained fine cellulose fiber dispersion was 1.0 mass%, the average fiber diameter of the fine cellulose fiber was 0.17 $\mu$m, and the average fiber length was 3 $\mu$m.

The obtained dispersion was subjected to solid-liquid separation treatment using a high-speed cooling centrifuge (manufactured by Hitachi Ltd., CR21G III) at 10 000 G for 20 minutes. The obtained precipitate was collected to obtain a dispersion (solid concentration: 12.0 mass%) of an anion-modified fine cellulose fiber.

[Manufacturing example A6: manufacture of short fiber (component (b))]

A mixture of 10.0 g of a carboxymethylated cellulose fiber (manufactured by Nippon Paper Industries Co., Ltd., SLD-F1, anionic group content: 1.7 mmol/g) and 300.0 g of a 1 M hydrochloric salt aqueous solution was stirred for 1 hour, and the solid content was collected by filtering with a filter paper. Deionized water was added over the solid content kept on the filter paper to wash the solid content. The washing was completed after it was confirmed with an electroconductivity meter (manufactured by HORIBA, Ltd., LAQUAtwin EC-33B) that the electric conductivity of the filtrate was 20 $\mu$S/cm or less. After completion of the washing, the solid content was left to stand until the filtrate stopped flowing to obtain a carboxymethylated cellulose fiber in which the content of the salt-type anionic group is 0% relative to the anionic group of the cellulose fiber. The solid content of the obtained fiber was 73 mass%.

A 1 M sodium hydroxide aqueous solution was added to a mixture of 30.0 g (absolute dry mass) of the carboxymethylated cellulose fiber obtained above and 900.0 g of deionized water such that the salt-type anionic group was 10 mol% relative to the anionic group of the cellulose fiber, and deionized water was then added thereto such that 1 000 g of a cellulose fiber aqueous solution was obtained, followed by stirring for 1 hour. Furthermore, refinement treatment was performed three times by using a mass-colloider (manufactured by Masuko Sangyo Co., Ltd., MKCA6-2) to obtain a carboxymethylated cellulose fiber dispersion (solid content: 3.0 mass%) in which the salt-type anionic group was 10 mol% relative to the anionic group of the cellulose fiber. The average fiber diameter of the obtained fine cellulose fiber dispersion was 0.34 $\mu$m, and the average fiber length was 5 $\mu$m.

[Examples 13 to 14: oil-in-water type emulsified composition]

Oil-in-water type emulsified compositions were formulated by using the cellulose fibers obtained in manufacturing examples A5 and A6 according to the prescriptions shown in Table 5. The network formability, film uniformity, and ultraviolet protection effect of these oil-in-water type emulsified compositions were evaluated as above. The oil-in-water type emulsified compositions of Examples 13 and 14 were excellent in the network formability, film uniformity, and ultraviolet protection effect. The results of the evaluation of the film uniformity and ultraviolet protection effect are shown in Table 5.

[Table 5]

| Component (mass%) | | Example 13 | Example 14 |
|---|---|---|---|
| Fiber | Manufacturing example No. | A5 | A6 |
| | Polymer type | Cellulose | Cellulose |
| | Average fiber diameter X ($\mu$m) | 0.17 | 0.34 |

(continued)

| Component (mass%) | | | Example 13 | Example 14 | |
|---|---|---|---|---|---|
| | | Average fiber length Y ($\mu$m) | | 3 | 5 |
| | | Aspect ratio Y/X | | 17.6 | 14.7 |
| | | (Average fiber diameter)$^2$/(fiber content)($\mu$m$^2$/mass%) (cumulative fiber length) | | 0.00723 | 0.02890 |
| | | Fiber content(mass%) | | 4 | 4 |
| Component (a) | UV absorber | Ethylhexyl methoxycinnamate (*3) | | 16 | 16 |
| | | Diethylaminohydroxybenzoyl hexyl benzoate (*4) | | - | - |
| | | Bis-ethylhexyloxyphenol methoxyphenyl triazine (*5) | | - | - |
| | | Ethylhexyl triazone (*6) | | - | - |
| | | C12-15 alkyl benzoate (*7) | | 2 | 2 |
| Water | | | | 76.91 | 76.91 |
| (Acrylates/C10-30 alkyl acrylate) crosspolymer (*8) | | | | 0.15 | 0.15 |
| Isoceteth-20 | | | | 0.5 | 0.5 |
| Preservative | | | | 0.34 | 0.34 |
| pH adjuster | | | | 0.1 | 0.1 |
| Total | | | | 100 | 100 |
| | | | | | |
| Evaluation | Film uniformity | | | 3 | 3 |
| | UV protection effect | | | 7.26 | 6.01 |

Reference Signs List

10: electrostatic spraying device

11: syringe

12: high voltage source

13: conducting collector

11a: cylinder

11b: piston

11c: capillary

**Claims**

**1.** A film-forming composition comprising components (a) and (b):

(a) a nonvolatile oil containing an ultraviolet absorber; and
(b) a fiber having an average fiber diameter of 0.1 $\mu$m or more and 7 $\mu$m or less in an amount of 0.5 mass% or more and 10 mass% or less in the film-forming composition, wherein

(average fiber diameter)$^2$/(fiber content) ($\mu$m$^2$/mass%) is 0.005 or more and 7 or less.

2. The film-forming composition according to claim 1, wherein a mass ratio of the ultraviolet absorber to a total of the nonvolatile oil is 0.1 or more and 1 or less.

3. The film-forming composition according to claim 1 or 2, wherein the component (b) is a fiber of a water-insoluble polymer.

4. The film-forming composition according to claim 3, wherein the water-insoluble polymer is one or more selected from the group consisting of a polyester resin and a (meth)acrylic resin.

5. The film-forming composition according to claim 3, wherein the water-insoluble polymer is a (meth)acrylic resin.

6. The film-forming composition according to any one of claims 1 to 5, wherein the component (b) has an average fiber length of 20 $\mu$m or more and 300 $\mu$m or less.

7. The film-forming composition according to any one of claims 1 to 6, wherein a content of the component (a) in the film-forming composition is 0.5 mass% or more and 90 mass% or less.

8. The film-forming composition according to any one of claims 1 to 7, wherein a content of the ultraviolet absorber in the film-forming composition is 0.1 mass% or more.

9. The film-forming composition according to any one of claims 1 to 8, wherein (average fiber diameter)$^2$/(fiber content) ($\mu$m$^2$/mass%) of the component (b) is 0.02 or more and 0.5 or less, the fiber of the component (b) has an aspect ratio, (average fiber length/average fiber diameter), of 15 or more, and the content of the ultraviolet absorber in the film-forming composition is 7 mass% or more.

10. The film-forming composition according to any one of claims 1 to 9, wherein (average fiber diameter)$^2$/(fiber content) ($\mu$m$^2$/mass%) of the component (b) is 0.02 or more and 0.5 or less, the fiber of the component (b) has an aspect ratio, (average fiber length)/(average fiber diameter), of 15 or more, and the mass ratio of the ultraviolet absorber to the total of the nonvolatile oil is 0.5 or more.

11. The film-forming composition according to any one of claims 1 to 10, wherein the composition is sunscreen.

12. A method for manufacturing a film on a skin surface, comprising applying the film-forming composition according to any one of claims 1 to 11 to the skin.

13. A film comprising the film-forming composition according to any one of claims 1 to 11.

[Figure 1]

[Figure 2]

[Figure 3]

[Figure 4]

[Figure 5]

## INTERNATIONAL SEARCH REPORT

| International application No. |
| --- |
| **PCT/JP2021/043804** |

**A. CLASSIFICATION OF SUBJECT MATTER**

*A61Q 17/00*(2006.01)i; *A61K 8/37*(2006.01)i; *A61K 8/41*(2006.01)i; *A61K 8/49*(2006.01)i; *A61K 8/72*(2006.01)i; *A61K 8/92*(2006.01)i
FI: A61K8/92; A61Q17/00; A61K8/72; A61K8/41; A61K8/49; A61K8/37

According to International Patent Classification (IPC) or to both national classification and IPC

**B. FIELDS SEARCHED**

Minimum documentation searched (classification system followed by classification symbols)

A61Q17/00; A61K8/37; A61K8/41; A61K8/49; A61K8/72; A61K8/92

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2020-105119 A (SANO SHOKAI KK) 09 July 2020 (2020-07-09) claims 1-5, paragraphs [0007], [0037], [0059]-[0061], [0072]-[0074] | 1-3, 6-13 |
| Y | claims 1-5, paragraphs [0007], [0037], [0059]-[0061], [0072]-[0074] | 4-5 |
| Y | JP 2020-090487 A (KAO CORP.) 11 June 2020 (2020-06-11) paragraphs [0013]-[0017] | 4-5 |
| A | JP 2020-63239 A (KAO CORP.) 23 April 2020 (2020-04-23) claim 1 | 1-13 |

☐ Further documents are listed in the continuation of Box C. ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **20 January 2022** | **15 February 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

International application No.

**PCT/JP2021/043804**

| Patent document cited in search report | | | Publication date (day/month/year) | Patent family member(s) | | | Publication date (day/month/year) |
|---|---|---|---|---|---|---|---|
| JP | 2020-105119 | A | 09 July 2020 | WO | 2020/138144 | A1 | |
| | | | | claims 1-5, paragraphs [0037], [0059]-[0061], [0072]-[0074] | | | |
| JP | 2020-090487 | A | 11 June 2020 | WO | 2020/111055 | A1 | |
| | | | | paragraphs [0013]-[0017] | | | |
| JP | 2020-63239 | A | 23 April 2020 | WO | 2020/067561 | A1 | |
| | | | | claim 1 | | | |
| | | | | CN | 112752564 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005320506 A
- JP 2002293731 A

**Non-patent literature cited in the description**

- **FUJITA.** *Kagaku no ryoiki (Journal of Japanese chemistry),* 1957, vol. 11 (10), 719-725